(19) Europäisches Patentamt
European Patent Office
Office européen des brevets

(11) Veröffentlichungsnummer: **0 155 566**
**A1**

(12) # EUROPÄISCHE PATENTANMELDUNG

(21) Anmeldenummer: 85102291.3

(22) Anmeldetag: 01.03.85

(51) Int. Cl.⁴: C 07 F 9/65
A 01 N 57/24
//C07D231/10

(30) Priorität: 13.03.84 DE 3409081

(43) Veröffentlichungstag der Anmeldung:
25.09.85 Patentblatt 85/39

(84) Benannte Vertragsstaaten:
CH DE FR GB IT LI

(71) Anmelder: BAYER AG
Konzernverwaltung RP Patentabteilung
D-5090 Leverkusen 1 Bayerwerk(DE)

(72) Erfinder: Pfister, Theodor, Dr.
Lichtenberger Strasse 30
D-4019 Monheim(DE)

(72) Erfinder: Eue, Ludwig, Dr.
Paul-Klee-Strasse 36
D-5090 Leverkusen 1(DE)

(72) Erfinder: Santel, Hans-Joachim, Dr.
Gerstenkamp 19
D-5000 Köln 80(DE)

(72) Erfinder: Schmidt, Robert R., Dr.
Im Waldwinkel 110
D-5060 Bergisch-Gladbach 2(DE)

(72) Erfinder: Hänssler, Gerd, Dr.
Heymannstrasse 40
D-5090 Leverkussen 1(DE)

(54) **Chlorierte Phosphorylmethylcarbonylpyrazol-Derivate.**

(57) Die Erfindung betrifft neue chlorierte Phosphorylmethylcarbonylpyrazol-Derivate der Formel (I)

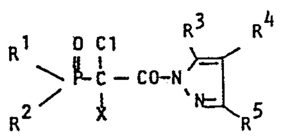

(I)

in welcher

$R^1$ für gegebenenfalls substituierte Reste aus der Reihe Alkyl, Aryl, Aralkyl, Alkoxy und Aralkoxy steht,

$R^2$ für gegebenenfalls substituierte Reste aus der Reihe Alkoxy oder Aralkoxy steht, oder

$R^1$ und $R^2$ gemeinsam für einen Alkandioxy-Rest stehen,

X für Wasserstoff oder Chlor steht,

$R^3$ und $R^5$ gleich oder verschieden sind und für Wasserstoff oder Alkyl stehen und

$R^4$ für Wasserstoff, Halogen oder Alkyl steht,

ein Verfahren zu ihrer Herstellung und ihre Verwendung als Pflanzenschutzmittel, insbesondere als Herbizide und Fungizide.

EP 0 155 566 A1

Croydon Printing Company Ltd.

BAYER AKTIENGESELLSCHAFT 5090 Leverkusen, Bayerwerk
Konzernverwaltung RP
Patentabteilung Bi/Em
Ia

# Chlorierte Phosphorylmethylcarbonylpyrazol-Derivate

Die Erfindung betrifft neue Phosphorylmethylcarbonylpyrazol-Derivate, ein Verfahren zu ihrer Herstellung und ihre Verwendung als Pflanzenschutzmittel, insbesondere als Herbizide und Fungizide.

Es ist bekannt, daß bestimmte chlorierte Phosphorylessigsäureester als Herbizide verwendet werden können (vgl. z. B. EP 1 018). So kann z. B. 2,2-Dichlor-2-(diethoxyphosphoryl)-essigsäure-(1-methyl)-ethylester zur Unkrautbekämpfung eingesetzt werden. Außerdem ist bekannt, daß bestimmte Bis-dithiocarbamate, wie z. B. Zinkethylen-bis-dithiocarbamat, fungizid wirksam sind (vgl. US-PS 2 457 674 und 3 050 439). Die Wirkung dieser Verbindungen ist jedoch insbesondere bei niedrigen Aufwandmengen und Konzentrationen nicht immer ausreichend.

Es wurden nun neue chlorierte Phosphorylmethylcarbonylpyrazol-Derivate der Formel(I) gefunden,

0155566

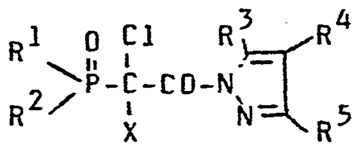

(I)

in welcher

$R^1$ für gegebenenfalls substituierte Reste aus der Reihe Alkyl, Aryl, Aralkyl, Alkoxy und Aralkoxy steht,

$R^2$ für gegebenenfalls substituierte Reste aus der Reihe Alkoxy oder Aralkoxy steht, oder

$R^1$ und $R^2$ gemeinsam für einen Alkandioxy-Rest stehen,

X für Wasserstoff oder Chlor steht,

$R^3$ und $R^5$ gleich oder verschieden sind und für Wasserstoff oder Alkyl stehen und

$R^4$ für Wasserstoff, Halogen oder Alkyl steht.

Man erhält die neuen Verbindungen der Formel (I), indem man Phosphorylmethylcarbonylpyrazol-Derivate der Formel (II)

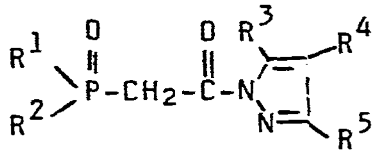

(II)

in welcher

$R^1$, $R^2$, $R^3$, $R^4$ und $R^5$ die oben angegebenen Bedeutungen haben,

mit einem Chlorierungsmittel gegebenenfalls in Gegenwart eines Säureakzeptors und gegebenenfalls in Gegenwart eines Verdünnungsmittels umsetzt.

Die neuen chlorierten Phosphorylmethylcarbonylpyrazol-Derivate der Formel (I) zeichnen sich durch hohe herbizide und fungizide Wirksamkeit aus.

Ueberraschenderweise sind die erfindungsgemäßen Wirkstoffe der Formel (I) gegen ökonomisch wichtige Ungräser in dikotylen Kulturen, wie Baumwolle und Soja den vorbekannten chlorierten Phosphorylessigsäureestern (gemäß EP 1 018) überlegen. Sie zeigen außerdem eine bessere systemische Wirkung gegen Pflanzenkrankheiten als die bereits erwähnten Bis-thiocarbamate (gemäß US-PS 2 457 674 und 3 050 439).

Gegenstand der Erfindung sind vorzugsweise die neuen chlorierten Phosphorylmethylcarbonylpyrazol-Derivaten der Formel (I), in welcher

$R^1$ für gegebenenfalls einfach oder mehrfach durch Halogen [wie insbesondere Fluor, Chlor und/oder Brom] oder $C_1$-$C_4$-Alkoxy substituierte Reste aus der Reihe Alkyl mit bis zu 6 Kohlenstoffatomen, Alkoxy mit bis zu 6 Kohlenstoffatomen, Aralkyl oder Aralkoxy mit 6 bis 10 Kohlenstoffatomen im Arylteil und bis zu 2 Kohlenstoffatomen im Alkylteil [wie insbesondere Benzyl, Phenylethyl, Benzyloxy und Phenylethoxy] oder für gegebenenfalls einfach oder mehrfach durch Halogen [wie insbesondere Fluor, Chlor und/oder Brom], Nitro, $C_1$-$C_4$-Alkyl, $C_1$-$C_4$-Alkoxy und/oder $C_1$-$C_4$-Alkoxycarbonyl substituiertes Phenyl steht,

$R^2$ für gegebenenfalls einfach oder mehrfach durch Halogen [wie insbesondere Fluor, Chlor und/oder Brom] oder

$C_1$-$C_4$-Alkoxy substituiertes Alkoxy mit bis zu 6 Kohlenstoffatomen und Aralkoxy mit 6 bis 10 Kohlenstoffatomen im Arylteil und bis zu 2 Kohlenstoffatomen im Alkylteil [wie insbesondere Benzyloxy oder Phenylethoxy] steht, oder

$R^1$ und $R^2$ gemeinsam für einen verzweigten oder unverzweigten Alkandioxy-Rest mit 2 bis 5 Kohlenstoffatomen im Alkylteil stehen,

X für Wasserstoff oder Chlor steht,

$R^3$ und $R^5$ gleich oder verschieden sind und für Wasserstoff oder $C_1$-$C_4$-Alkyl stehen und

$R^4$ für Wasserstoff, Halogen [wie insbesondere Chlor] oder $C_1$-$C_4$-Alkyl steht.

Die Erfindung betrifft insbesondere Verbindungen der Formel (I), in welcher

$R^1$ für Ethoxy, n-Propoxy, i-Propoxy, n-Butoxy, i-Butoxy, sec.-Butoxy, tert.-Butoxy oder Phenyl steht,

$R^2$ für Ethoxy, n-Propoxy, i-Propoxy, n-Butoxy, i-Butoxy, sec.-Butoxy oder tert.-Butoxy steht,

X für Wasserstoff oder Chlor steht und

$R^3$ und $R^5$ gleich oder verschieden sind und für Wasserstoff oder Methyl stehen und

$R^4$ für Wasserstoff oder Chlor steht.

Verwendet man beispielsweise Natriumhypochlorit als Chlorierungsmittel und 1-Di-propoxy-phosphoryl-methylcarbonylpyrazol als Ausgangsstoff, so kann der Reaktionsablauf nach dem erfindungsgemäßen Verfahren durch folgendes Formelschema wiedergegeben werden:

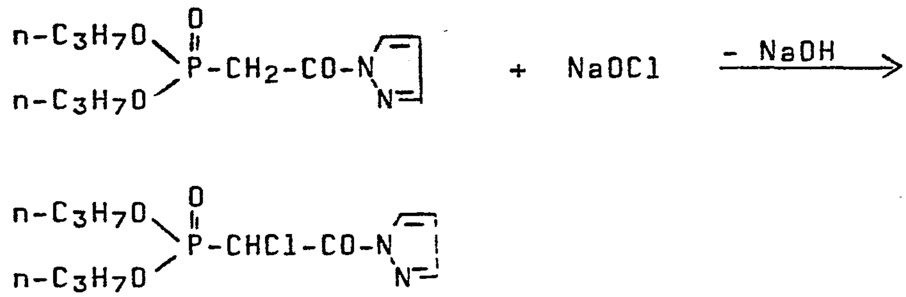


Die als Ausgangsstoffe zu verwendenden Phosphorylmethylcarbonylpyrazol-Derivate sind durch die Formel (II) definiert. In dieser Formel stehen $R^1$, $R^2$, $R^3$, $R^4$ und $R^5$ vorzugsweise bzw. besonders bevorzugt für diejenigen Reste, welche bereits im Rahmen der Substituentendefinitionen der Formel (I) vorzugsweise bzw. als besonders bevorzugt genannt sind.

Als Ausgangsstoffe für die Verbindungen der Formel (II) seien beispielsweise genannt:

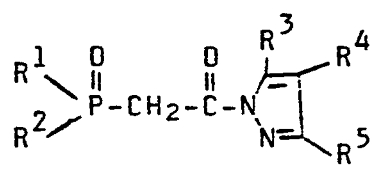
(II)

Tabelle 1

| $R^1$ | $R^2$ | $R^3$ | $R^4$ | $R^5$ |
|---|---|---|---|---|
| $OC_2H_5$ | $OC_2H_5$ | $CH_3$ | H | $CH_3$ |
| $OCH(CH_3)_2$ | $OCH(CH_3)_2$ | H | H | H |
| $OCH(CH_3)_2$ | $OCH(CH_3)_2$ | $CH_3$ | H | $CH_3$ |
| $OC_2H_5$ | $OC_2H_5$ | H | H | H |
| $OC_2H_5$ | $OC_2H_5$ | H | Cl | H |
| $OC_2H_5$ | $OC_2H_5$ | $CH_3$ | Cl | $CH_3$ |
| $OC_2H_5$ | $OC_2H_5$ | $CH_3$ | $CH_3$ | $CH_3$ |
| $OC_3H_7$ | $OC_3H_7$ | H | Cl | H |
| $OC_3H_7$ | $OC_3H_7$ | $CH_3$ | Cl | H |
| $OC_3H_7$ | $OC_3H_7$ | $CH_3$ | $CH_3$ | $CH_3$ |

Die Ausgangsstoffe der Formel (II) sind zum Teil noch nicht bekannt. Man erhält diese Verbindungen, wenn man Halogenmethylcarbonylpyrazol-Derivate der Formel (III)

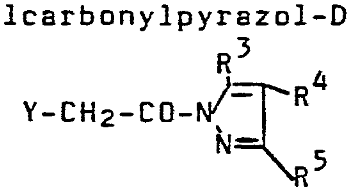

(III)

in welcher

$R^3$, $R^4$ und $R^5$ die oben angegebenen Bedeutungen haben und

Y für Halogen, insbesondere für Chlor oder Brom steht,

mit Verbindungen der Formel (IV)

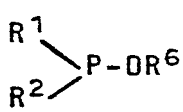

(IV)

in welcher

$R^1$ und $R^2$ die oben genannten Bedeutungen haben und

$R^6$ für gegebenenfalls substituiertes $C_1$-$C_4$-Alkyl oder Benzyl steht,

auf Temperaturen zwischen 50 °C und 200 °C, vorzugsweise zwischen 100 °C und 150 °C erhitzt und die Reaktionsprodukte der Formel Y-$R^6$, gegebenenfalls unter vermindertem Druck entfernt.

Die Halogenmethylcarbonylpyrazol-Derivate der Formel (III) sind zum Teil noch nicht in der Literatur beschrieben. Man erhält diese Verbindungen, wenn man Halogenessigsäurehalogenide der Formel (V)

$$Y-CH_2-CO-Y^1 \qquad (V)$$

in welcher

Y und $Y^1$ gleich oder verschieden sind und für Halogen, insbesondere für Chlor oder Brom stehen,

mit Pyrazolen der Formel (VI)

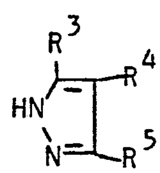

(VI)

in welcher

$R^3$, $R^4$ und $R^5$ die oben angegebenen Bedeutungen haben,

in Gegenwart von Säureakzeptoren, wie z. B. Triethylamin, und in Gegenwart von Verdünnungsmitteln, wie z. B. Methylenchlorid, bei Temperaturen zwischen -50 °C und +100 °C, vorzugsweise zwischen -20 °C und +50 °C umsetzt.

Die Aufarbeitung kann nach üblichen Methoden erfolgen, beispielsweise durch Waschen der organischen Lösung mit wäßriger Natriumhydrogencarbonat-Lösung, Trocknen, Filtrieren der organischen Phase und Abdestillieren des Lösungsmittels unter vermindertem Druck.

Die Verbindungen der Formel (IV) sind allgemein bekannte Verbindungen der organischen Chemie.

Als Beispiele seien genannt: Phosphorigsäure-tri-ethylester, -tri-n-propylester, -tri-i-propylester, tri-n-butylester, tri-i-butylester und -tri-sec.-butylester sowie Phenylphosphonigsäure-di-ethylester, -di-n-propylester, di-i-propylester, -di-n-butylester, -di-i-butylester und -di-sec.-butylester.

Die Ausgangsstoffe der Formeln (V) und (VI) sind allgemein bekannte Verbindungen der organischen Chemie.

Das Verfahren zur Herstellung der neuen Verbindungen der Formel (I) wird unter Verwendung von Chlorierungsmitteln durchgeführt. Als solche sind Chlor, Sulfurylchlorid und Alkalimetall- oder Erdalkalimetall-hypochlorite, wie z. B. Natriumhypochlorit (gegebenenfalls in Form der sogenannten Chlorlauge) oder Calcium-hypochlorit zu nennen. Vorzugsweise wird Chlorlauge verwendet.

Das erfindungsgemäße Verfahren wird vorzugsweise in Gegenwart von Verdünnungsmitteln durchgeführt. Als solche kommen insbesondere halogenierte Kohlenwasserstoffe, wie z. B. Methylenchlorid, Ethylenchlorid, Chloroform oder Tetrachlormethan in Betracht. Bei Einsatz von Chlorlauge wird Wasser als Verdünnungsmittel verwendet, das gegebenenfalls durch ein inertes organisches Lösungsmittel ergänzt wird.

Beim erfindungsgemäßen Verfahren werden gegebenenfalls Säureakzeptoren verwendet. Bei Einsatz von wäßrigen Hypochlorit-Lösungen dienen vorzugsweise entsprechende Alkalihydroxide oder Erdalkalihydroxide, wie z. B. Natrium-, Kalium- oder Calcium-hydroxid, als Säurebindemittel.

Die Reaktionstemperaturen können beim erfindungsgemäßen Verfahren innerhalb eines größeren Bereiches variiert werden. Im allgemeinen arbeitet man zwischen -20 °C und +50 °C, vorzugsweise bei 0 °C bis 30 °C. Das Verfahren wird im allgemeinen bei Normaldruck durchgeführt.

Zur Durchführungdes erfindungsgemäßen Verfahrens werden im allgemeinen je Mol Phosphorylmethylcarbonylpyrazol-Derivat der Formel (II) zwischen 0,5 und 5, vorzugsweise zwischen 0,9 und 3,5 Moläquivalente Chlorierungsmittel eingesetzt und der pH-Wert wird im allgemeinen zwischen 7 und 14, vorzugsweise zwischen 9 und 14 gehalten.

Im allgemeinen werden Chlorierungsmittel und das Verdünnungsmittel vorgelegt und die Verbindung der Formel (II) wird unter starkem Rühren dazugegeben. Nach Ende der Umsetzung kann nach üblichen Methoden aufgearbeitet werden, beispielsweise durch Waschen der organischen Lösung mit wäßriger Natriumhydrogencarbonatlösung, Trocknen und Filtrieren der organischen Phase und Abdestillieren des Lösungsmittels unter vermindertem Druck bei mäßig erhöhter Temperatur. Die schwerflüchtigen Rückstände enthalten im

wesentlichen die Verbindungen der Formel (I), welche durch ihre physikalisch-chemischen Eigenschaften charakterisiert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe können selektiv zur Gräserbekämpfung in Kulturpflanzen wie z. B. Baumwolle und Sojabohnen eingesetzt werden.

Die erfindungsgemäßen Wirkstoffe weisen außerdem eine stark mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können die erfindungsgemäßen Wirkstoffe mit

besonders gutem Erfolg zur systemischen Bekämpfung von Reiskrankheiten, wie Pyricularia oryzae eingesetzt werden.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowieULV-Formulierungen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch als Mischungen mit bekannten Herbiziden zur Unkräutbekämpfung Verwendung finden, wobei Fertigformulierung oder Tankmischung möglich ist.

Für die Mischungen kommen bekannte Herbizide wie zum Beispiel 1-Amino-6-ethylthio-3-(2,2-dimethylpropyl)-1,3,5-triazin-2,4-(1H, 3H)-dion oder N-(2-Benzthiazolyl)-N,N'-dimethyl-harnstoff zur Unkrautbekämpfung in Getreide, 4-Amino-3-methyl-6-phenyl-1,2,4-triazin-5-(4H)-on zur Unkrautbekämpfung in Zuckerrüben und 4-Amino-6-(1,1-dimethylethyl)-3-methylthio-1,2,4-triazin-5-(4H)-on zur Unkrautbekämpfung in Sojabohnen infrage. Einige dieser Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z. B. durch Gießen, Spritzen, Sprühen, Streuen, Tauchen, Vernebeln, Verdampfen, Injizieren, Verschlämmen, Verstreichen, Stäuben, Trockenbeizen, Feuchtbeizen, Naßbeizen, Schlämmbeizen oder Inkrustieren.

Die erfindungsgemäßen Wirkstoffe können bei Herbizid-Anwendung sowohl vor als auch nach dem Auflaufen der Pflanzen appliziert werden. Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effektes ab. Im allgemeinen liegen die Aufwandmengen zwischen 0,05 und 10 kg Wirkstoff pro ha, vorzugsweise zwischen 0,1 und 5 kg pro ha.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise 0,0001 bis 0,02 Gew.-%, am Wirkungsort erforderlich.

Die nachfolgenden Beispiele dienen zur weiteren Erläuterung der Erfindung.

Herstellungsbeispiele

Beispiel 1

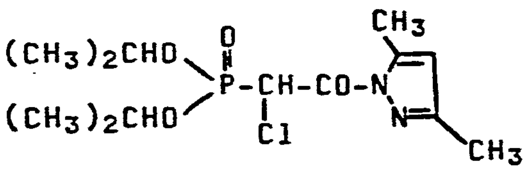

Eine Lösung von 10 g (0,033 Mol) 1-(Diisopropoxyphosphorylmethylcarbonyl)-3,5-dimethyl-pyrazol in 50 ml Tetrachlormethan wurde unter Rühren zu einer auf 0 °C bis 5 °C gekühlten Mischung aus 37,6 g 11,5 %iger Chlorlauge und 100 ml Tetrachlormethan tropfenweise gegeben und das Reaktionsgemisch wurde zwei Stunden bei 0 °C bis 5 °C gerührt. Die organische Phase wurde getrennt, mit eiskalter Natriumhydrogencarbonatlösung gewaschen, mit Natriumsulfat getrocknet, und filtiert. Vom Filtrat wurde das Lösungsmittel unter vermindertem Druck sorgfältig abdestilliert.

Man erhielt 5,2 g (47 % der Theorie) 1-(Diisopropoxyphosphorylchlormethylcarbonyl)-3,5-dimethyl-pyrazol als gelbes Öl vom Brechungsindex $n_D^{20}$ = 1,4574.

Analog Beispiel 1 konnten die in der nachstehenden Tabelle 2 aufgeführten Verbindungen der Formel (I) hergestellt werden:

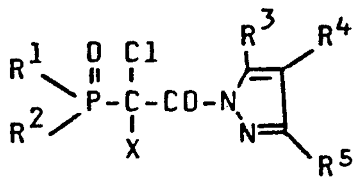

(I)

Tabelle 2

| Beisp. Nr. | $R^1$ | $R^2$ | X | $R^3$ | $R^4$ | $R^5$ | Brechungsindex $n_D^{20}$: |
|---|---|---|---|---|---|---|---|
| 2 | $-OCH(CH_3)_2$ | $-OCH(CH_3)_2$ | Cl | H | H | H | 1,5095 |
| 3 | $-OCH(CH_3)_2$ | $-OCH(CH_3)_2$ | Cl | $CH_3$ | H | $CH_3$ | 1,4786 |
| 4 | $-OC_2H_5$ | $-OC_2H_5$ | Cl | $CH_3$ | H | $CH_3$ | |
| 5 | $-OCH(CH_3)_2$ | $-OCH(CH_3)_2$ | H | $CH_3$ | Cl | $CH_3$ | |
| 6 | $-OCH(CH_3)_2$ | $-OCH(CH_3)_2$ | H | H | Cl | H | |
| 7 | $-OC_2H_5$ | $-OC_2H_5$ | Cl | H | H | H | |
| 8 | $-OC_2H_5$ | $-OC_2H_5$ | H | $CH_3$ | $CH_3$ | $CH_3$ | |
| 9 | $-OCH(CH_3)_2$ | $-OCH(CH_3)_2$ | H | $CH_3$ | $CH_3$ | $CH_3$ | |
| 10 | $-OCH(CH_3)_2$ | $-OCH(CH_3)_2$ | Cl | $CH_3$ | $CH_3$ | $CH_3$ | |
| 11 | $-OC_2H_5$ | $-OC_2H_5$ | Cl | $CH_3$ | $CH_3$ | $CH_3$ | |

Herstellung von Ausgangsstoffen der Formel (II)

Beispiel II-1

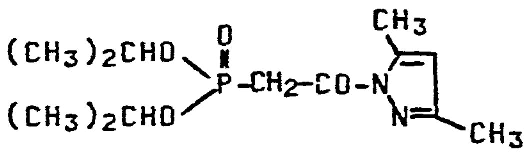

18,4 g (0,09 Mol) Triisopropylphosphit wurden bei ca. 50 °C zu 13,8 g (0,08 Mol) 1-Chlormethylcarbonyl-3,5-dimethyl-pyrazol gegeben und das Reaktionsgemisch wurde unter Rühren 4 Stunden auf 140 °C erhitzt. Anschließend wurden flüchtige Komponenten unter vermindertem Druck abdestilliert.

Man erhielt 23,7 g (98 % der Theorie) 1-(Diisopropoxyphosphorylmethylcarbonyl)-3,5-dimethyl-pyrazol als Oel vom Brechungsindex $n_D^{20} = 1,4645$.

Analog konnten die in der nachstehenden Tabelle 3 aufgeführten Verbindungen der Formel (II) hergestellt werden:

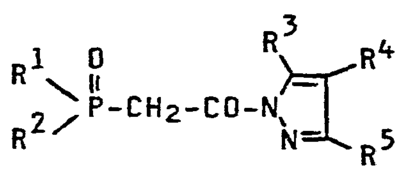

(II)

Tabelle 3

| Beisp. Nr. | $R^1$ | $R^2$ | $R^3$ | $R^4$ | $R^5$ | Brechungs-index $n_D^{20}$: |
|---|---|---|---|---|---|---|
| II-2 | $-OCH(CH_3)_2$ | $-OCH(CH_3)_2$ | H | H | H | 1,4700 |
| II-3 | $-OC_2H_5$ | $-OC_2H_5$ | H | H | H | |
| II-4 | $-OC_2H_5$ | $-OC_2H_5$ | $CH_3$ | H | $CH_3$ | |
| II-5 | $-OC_2H_5$ | $-OC_2H_5$ | H | Cl | H | |
| II-6 | $-OC_2H_5$ | $-OC_2H_5$ | $CH_3$ | Cl | $CH_3$ | |
| II-7 | $-OC_2H_5$ | $-OC_2H_5$ | $CH_3$ | $CH_3$ | $CH_3$ | |
| II-8 | $-OCH(CH_3)_2$ | $-OCH(CH_3)_2$ | H | Cl | H | |
| II-9 | $-OCH(CH_3)_2$ | $-OCH(CH_3)_2$ | $CH_3$ | Cl | $CH_3$ | |

Herstellung von Ausgangsstoffen der Formel (III)

Beispiel III-1

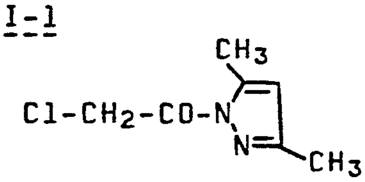

22,4 g (0,2 Mol) Chloracetylchlorid wurden unter Rühren bei 30 °C zu einer Mischung aus 19,2 g (0,2 Mol) 3,5-Dimethylpyrazol, 20,4 g (0,2 Mol) Triethylamin und 50 ml Methylenchlorid gegeben. Das Reaktionsgemisch wurde ca. 15 Stunden bei 15 °C bis 25°C gerührt, mit Natriumhydrogencarbonatlösung gewaschen, mit Natriumsulfat getrocknet, filtiert und vom Filtrat wurden unter vermindertem Druck flüchtige Komponenten abdestilliert, wobei das Produkt kristallisierte.

Man erhielt 28,4 g (82 % der Theorie) 1-Chlormethylcarbonyl-3,5-dimethyl-pyrazol vom Schmelzpunkt 62 °C.

Analog konnten die in der nachstehenden Tabelle 4 aufgeführten Verbindungen der Formel (III) hergestellt werden:

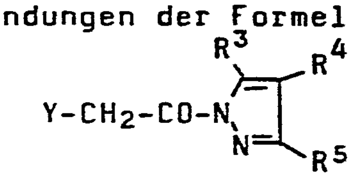

(III)

Tabelle 4

| Beisp. Nr. | Y | $R^3$ | $R^4$ | $R^5$ | Brechungsindex $n_D^{20}$: |
|---|---|---|---|---|---|
| III-2 | Cl | H | H | H | 1,5289 |
| III-3 | Br | H | H | H | |
| III-4 | Br | $CH_3$ | H | $CH_3$ | |
| III-5 | Cl | $CH_3$ | Cl | $CH_3$ | |
| III-6 | Cl | $CH_3$ | $CH_3$ | $CH_3$ | |
| III-7 | Cl | H | Cl | H | |

Beispiel A

Pre-emergence-Test / Gewächshaus

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigt z. B. die folgende Verbindung gemäß den Herstellungsbeispielen eine ausgezeichnete Wirksamkeit: (1).

Beispiel B

Pyricularia-Test (Reis) /systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25 °C und einer rel. Luftfeuchtigkeit von 100 % bis zur Auswertung.

4 Tage nach der Inokulation erfolgt die Auswertung des Kankheitsbefalls.

Eine deutliche Ueberlegenheit in der Wirksamkeit gegenüber dem Stand der Technik zeigt in diesem Test z. B. die Verbindung gemäß dem folgenden Herstellungsbeispiel:
(3)

0155566

Patentansprüche

1. Chlorierte Phosphorylmethylcarbonylpyrazol-Derivate der allgemeinen Formel (I)

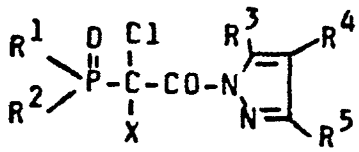

(I)

in welcher

$R^1$ für gegebenenfalls substituierte Reste aus der Reihe Alkyl, Aryl, Aralkyl, Alkoxy und Aralkoxy steht,

$R^2$ für gegebenenfalls substituierte Reste aus der Reihe Alkoxy oder Aralkoxy steht, oder

$R^1$ und $R^2$ gemeinsam für einen Alkandioxy-Rest stehen,

X für Wasserstoff oder Chlor steht,

$R^3$ und $R^5$ gleich oder verschieden sind und für Wasserstoff oder Alkyl stehen und

$R^4$ für Wasserstoff, Halogen oder Alkyl steht.

2. Chlorierte Phosphorylmethylcarbonylpyrazol-Derivate der allgemeinen Formel (I) gemäß Anspruch 1, dadurch gekennzeichnet, daß darin

$R^1$ für gegebenenfalls einfach oder mehrfach durch Halogen oder $C_1$-$C_4$-Alkoxy substituierte Reste aus der Reihe Alkyl mit bis zu 6 Kohlenstoffatomen, Alkoxy mit bis zu 6 Kohlenstoffatomen, Aralkyl oder Aralkoxy mit 6 bis 10 Kohlenstoffatomen im Arylteil und bis zu 2 Kohlenstoffatomen im Alkylteil oder für gegebenenfalls einfach oder mehrfach durch Halogen, Nitro, $C_1$-$C_4$-Alkyl, $C_1$-$C_4$-Alkoxy und/oder $C_1$-$C_4$-Alkoxycarbonyl substituiertes Phenyl steht,

$R^2$ für gegebenenfalls einfach oder mehrfach durch Halogen oder $C_1$-$C_4$-Alkoxy substituiertes Alkoxy mit bis zu 6 Kohlenstoffatomen und Aralkoxy mit 6 bis 10 Kohlenstoffatomen im Arylteil und bis zu 2 Kohlenstoffatomen im Alkylteil steht, oder

$R^1$ und $R^2$ gemeinsam für einen verzweigten oder unverzweigten Alkandioxy-Rest mit 2 bis 5 Kohlenstoffatomen im Alkylteil stehen,

X für Wasserstoff oder Chlor steht,

$R^3$ und $R^5$ gleich oder verschieden sind und für Wasserstoff oder $C_1$-$C_4$-Alkyl stehen und

$R^4$ für Wasserstoff, Halogen oder $C_1$-$C_4$-Alkyl steht.

3. Chlorierte Phosphorylmethylcarbonylpyrazol-Derivate der allgemeinen Formel (I) gemäß Anspruch 1, dadurch gekennzeichnet, daß darin

$R^1$ für Ethoxy, n-Propoxy, i-Propoxy, n-Butoxy, i-Butoxy, sec.-Butoxy, tert.-Butoxy oder Phenyl steht,

$R^2$ für Ethoxy, n-Propoxy, i-Propoxy, n-Butoxy, i-Butoxy, sec.-Butoxy oder tert.-Butoxy steht,

X für Wasserstoff oder Chlor steht und

$R^3$ und $R^5$ gleich oder verschieden sind und für Wasserstoff oder Methyl stehen und

$R^4$ für Wasserstoff oder Chlor steht.

4. Verfahren zur Herstellung von chlorierten Phosphorylmethylcarbonylpyrazol-Derivaten der Formel (I) gemäß Anspruch 1, dadurch gekennzeichnet, daß man Phosphorylmethylcarbonylpyrazol-Derivate der Formel (II)

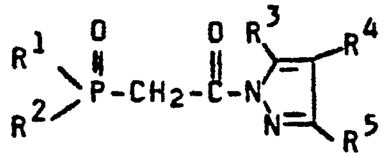

(II)

in welcher

$R^1$, $R^2$, $R^3$, $R^4$ und $R^5$ die in Anspruch 1 angegebenen Bedeutungen haben,

mit einem Chlorierungsmittel gegebenenfalls in Gegenwart eines Säureakzeptors und gegebenenfalls in Gegenwart eines Verdünnungsmittels umsetzt.

5. Herbizide Mittel, gekennzeichnet durch einen Gehalt an mindestens einem chlorierten Phosphorylmethylcarbonyl-pyrazol-Derivat der allgemeinen Formel (I) gemäß Anspruch 1.

6. Verwendung von chlorierten Phosphorylmethylcarbonylpyrazol-Derivaten der allgemeinen Formel (I) gemäß Anspruch 1 zur Bekämpfung von Unkraut.

7. Fungizide Mittel, gekennzeichnet durch einen Gehalt an mindestens einem chlorierten Phosphorylmethylcarbonylpyrazol-Derivat der allgemeinen Formel (I) gemäß Anspruch 1.

8. Verwendung von chlorierten Phosphorylmethylcarbonylpyrazol-Derivaten der allgemeinen Formel (I) gemäß Anspruch 1 zur Bekämpfung von unerwünschten Mikroorganismen.

9. Verfahren zur Herstellung von herbiziden bzw. fungiziden Mitteln, dadurch gekennzeichnet, daß man chlorierte Phosphorylmethylcarbonylpyrazol-Derivate der allgemeinen Formel (I) gemäß Anspruch 1 mit Streckmitteln und/oder oberflächenaktiven Mitteln vermischt.

0155566

Europäisches Patentamt

# EUROPÄISCHER RECHERCHENBERICHT

Nummer der Anmeldung

EP 85 10 2291

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | CH-A- 310 406 (CIBA AG)<br>* Insgesamt *<br>--- | 1-3,5-9 | C 07 F 9/65<br>A 01 N 57/24 //<br>C 07 D 231/10 |
| A | EP-A-0 011 363 (STAUFFER CHEMICAL CO.)<br>* Seiten 1-5; Seiten 12-17, Verbindungen 9,11,20,22,24,31 *<br>--- | 1-3,5-9 | |
| P,Y | EP-A-0 126 249 (BAYER AG)<br>* Ansprüche *<br>----- | 1-9 | |
| | | | RECHERCHIERTE SACHGEBIETE (Int. Cl.4) |
| | | | C 07 F 9/00 |

Der vorliegende Recherchenbericht wurde für alle Patentansprüche erstellt.

| Recherchenort | Abschlußdatum der Recherche | Prüfer |
|---|---|---|
| DEN HAAG | 07-06-1985 | BESLIER L.M. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82